# EUROPEAN PATENT SPECIFICATION

(11) **EP 0 932 408 B1**
(45) Date of publication and mention of the grant of the patent: **02.01.2002**
(21) Application number: 97911196.0
(22) Date of filing: 08.10.1997
(51) Int. Cl.: A61K 35/78, A61K 7/00

(54) **PHARMACEUTICAL AND COSMETIC FORMULATIONS WITH ANTIMICROBIAL ACTIVITY**
PHARMAZEUTISCHE UND KOSMETISCHE ZUBEREITUNGEN MIT ANTIMIKROBIELLER WIRKUNG
FORMULATIONS PHARMACEUTIQUES ET COSMETIQUES POSSEDANT UNE ACTIVITE ANTIMICROBIENNE

(30) Priority: 17.10.1996 IT MI962148
(43) Date of publication of application: 04.08.1999
(73) Proprietor: INDENA S.p.A., 20139 Milano (IT)
(72) Inventor: BOMBARDELLI, Ezio, I-20141 Milano (IT); MORAZZONI, Paolo, I-20139 Milano (IT)
(74) Representative: Minoja, Fabrizio, Dr.
(86) International application number: EP9705529
(87) International publication number: WO9817294

(56) References cited:
- EP-A- 0 464 297
- GB-A- 2 184 353
- IN-A- 139 868
- PATENT ABSTRACTS OF JAPAN vol. 013, no. 079 (C-571), 22 February 1989 & JP 63 267714 A (SATO SEIYAKU KK), 4 November 1988,

## Description

The present invention relates to pharmaceutical or cosmetic formulations with antimicrobial activity containing a) a hydrophilic extract of Krameria sp., or a pure compound from said extract, and b) a lipophilic extract of Mesua ferrea.

### PRIOR ART

It is known from literature (Martindale, "The extrapharmacopeia", 28th Ed. (1982); Cannizaro, Boll. Soc. Ital. Biol Sperim. 1,22, 1964; V. Hoppe, Drogenkunde Bdl Walter De Gruyter Ed., 1975; and British patent 2.184.353 A) that the extracts obtained by extraction with chlorinated solvents, aliphatic ethers and ketones as well as aliphatic and aromatic esters, from the roots, the bark of the trunk and the leaves of different species of Krameria, preferably Krameria triandra, enriched in neolignanes (particularly Eupomatenoid 6 and 2-(2,4-dihydroxyphenyl)-5-propenylbenzofuran) have antimicrobial activity against gram +, gram - bacteria, fungi and anaerobic strains.

These extracts, although obtainable by extraction even with protic solvents, are naturally hydrophilic due to the phenol character of their active components.

The pure components Eupomatenoid 6 and 2-(2,4-dihydroxyphenyl)-5-propenylbenzofuran can be recovered from these extracts using chromatographic techniques on silica gel, as reported in EP 0 464 297 B1.

From the flowers, particularly from the buds, of Mesua ferrea, extraction with aprotic solvents such as hexane, methylene chloride or better with carbon dioxide in hyper-critic conditions, for example extracting the vegetable material under pressures ranging from 110 to 260 bar, mainly at 200 bar and at temperatures ranging from 35 to 65°C, preferably at 45°C, yields a lipophilic extract containing substituted coumarins and xanthones, which results particularly active on gram +, gram - and anaerobic bacterial strains, with an activity comparable to that of the extract prepared from Krameria triandra.

### DISCLOSURE OF THE INVENTION

The present invention relates to a combination comprising:
- a hydrophilic extract of Krameria sp., preferably Krameria triandra, or a pure compound from said extract, and
- a lipophilic extract of Mesua ferrea, having antimicrobial activity.

"Pure compound from an extract of Krameria" means neolignanes such as Eupomatenoid 6 or 2-(2,4-dihydroxyphenyl)-5-propenylbenzofuran).

It has surprisingly been found that said combination, in ratios ranging from 1:10 to 10:1, preferably in a 1:1 ratio, leads to a synergism of the antimicrobial activity of the single extracts unforeseeable in advance.

The combinations of the present invention can be used also as preservatives in the alimentary and cosmetic industries. Therefore the present invention also relates to alimentary or cosmetic formulations containing as preservatives:
a) a hydrophilic extract of Krameria sp., preferably Krameria triandra, or a pure compound from said extract, and
b) a lipophilic extract of Mesua ferrea.

The invention relates as well to the use of:
a) a hydrophilic extract of Krameria sp., preferably Krameria triandra, or a pure compound from said extract, and
b) a lipophilic extract of Mesua ferrea,
as preservative for alimentary and cosmetic formulations.

The following table shows the average values of the in vitro antimicrobial activity of two exemplary extracts, respectively prepared from Krameria triandra, from Mesua ferrea, and those resulting from a combination in a 1:1 ratio.

Said activity, expressed in M.I.C., has been evaluated in agreement with the conventional procedures known to those skilled in the art.

**Table -**

| *In vitro* antimicrobial activity of the standardized extracts of Krameria triandra and Mesua ferrea. | | | | |
|---|---|---|---|---|
| Microbial strains | M.I.C. ug/ml | | | |
| | I | II | III | IV |
| Staphilococcus aureus MPR5 | 6.1 | 4 | 2 | 1 |
| Staphilococcus aureus ATCC6538 | 12 | 7 | 3 | 0.5 |
| Staphilococcus aureus F2 ISF 3 | 9 | 15 | 4 | - |
| Staphilococcus epidermis | | | | |
| HCF Berset C | 10 | 8 | 4 | - |
| Staphilococcus epidermis CPHL | 8 | 6 | 3 | 1 |
| Streptococcus feaecalis LEP Br | 10 | 6 | 2 | 16 |
| | | | | |
| Escherichia coli CNUR 1 | >128 | >128 | 32 | 0.51 |
| Acinetobacter cal. OSMPV | 6.2 | 4 | 2 | 2 |
| Pseudomonas aeruginosa CNUR 4 | 6 | 4.3 | 1 | 32 |
| Trichophyton mentagrophytes 193 | 128 | 61 | 12 | 2 |
| Candida albicans ATCC 10231 | 62.5 | 128 | 12 | 2 |
| Bacteroides fragilis | 26 | 16 | 4 | 32 |
| Propionibacterium acnes | 4 | 4 | 1 | 32 |
| I = extract of Krameria triandra prepared according to example II | | | | |
| II = extract of Mesua ferrea prepared according to example I | | | | |
| III= 1:1 combination of Krameria triandra and Mesua ferrea | | | | |
| IV = rufloxacin for bacterial, miconazole for fungi. | | | | |

The present invention, therefore, relates to pharmaceutical or cosmetic formulations with antimicrobial activity, which can be administered topically (gels, creams, lotions, milks and solid preparations) containing the combinations described above. Said formulations will be prepared according to conventional methods well known in pharmaceutical technique, as those described in "Remington's Pharmaceutical Handbook", Mack Publishing Co., N.Y., USA, together with suitable excipients, in particular antioxidants.

The following examples illustrate the invention in further detail.

### Example I - Preparation of the lipophilic extract of Mesua ferrea

1 kg of finely ground buds of Mesua ferrea is extracted in a 5 1 extractor with CO₂ in hyper-critic conditions; a first extraction is carried out at 34°C and 90 bars of pressure, using about 25 1 of carbon dioxide; the resulting extract is discarded and the vegetable material is subjected to a second extraction, increasing temperature to 45°C and pressure to 220 bar. 15.6 g of a very thick oil of orange colour are obtained. This oil can be used directly as such or subjected to further fractionations by means of conventional chemical separations.

### Example II - Preparation of the extract of Krameria triandra

2 kg of bark of the root of Krameria triandra, after grinding, are extracted three times with 5 1 each of acetone; the combined extracts are concentrated to small volume and the concentrate is taken up in 0.8 1 of acetone:water 1:1 (v/v). The resulting suspension is extracted twice with 0.5 1 of methylene chloride, the chloromethylene phase is dried over anhydrous sodium sulfate, then concentrated to dryness. 85 g of a reddish solid are obtained, containing about 26% of Eupomatenoid 6 and 14% of 2-(2,4-dihydroxyphenyl)-5-propenylbenzofuran.

### Example III - Preparation of the extract of Mesua ferrea with hexane

1 kg of finely ground buds of Mesua ferrea is extracted 4 times with 3 1 of hexane under reflux; the combined hexane extracts are decolourized with active charcoal (5 g) and concentrated under vacuum to an oil. 14.5 g of extract are obtained, which can be used without further fractionation.

## Claims

1. Pharmaceutical or cosmetic formulations with antimicrobial activity, containing:
a) a hydrophilic extract of Krameria sp., preferably Krameria triandra, or a pure compound from said extract selected from the group consisting of Eupomatenoid 6 or 2-(2,4-dihydroxyphenyl)-5-propenylbenzofuran, and
b) a lipophilic extract of Mesua ferrea.

2. Pharmaceutical formulations according to claim 1, in which the ratio of extracts of Krameria to extracts of Mesua ferrea ranges from 1:10 to 10:1.

3. Formulations according to claim 2, in which the ratio is 1:1.

4. The use of:
a) a hydrophilic extract of Krameria sp., preferably Krameria triandra, or a pure compound from said extract selected from the group consisting of Eupomatenoid 6 or 2-(2,4-dihydroxyphenyl)-5-propenylbenzofuran, and
b) a lipophilic extract of Mesua ferrea,
for the preparation of a medicament with antimicrobial activity.

5. Alimentary or cosmetic formulations containing as preservatives:
a) a hydrophilic extract of Krameria sp., preferably Krameria triandra, or a pure compound from said extract selected from the group consisting of Eupomatenoid 6 or 2-(2,4-dihydroxyphenyl)-5-propenylbenzofuran, and
b) a lipophilic extract of Mesua ferrea.

6. The use of:
a) a hydrophilic extract of Krameria sp., preferably Krameria triandra, or a pure compound from said extract selected from the group consisting of Eupomatenoid 6 or 2-(2,4-dihydroxyphenyl)-5-propenylbenzofuran, and
b) a lipophilic extract of Mesua ferrea,
as preservative for alimentary or cosmetic formulations.

## Patentansprüche

1. Pharmazeutische oder kosmetische Zubereitungen mit antimikrobieller Wirkung, enthaltend
a) einen hydrophilen Extrakt von Krameria sp., vorzugsweise von Krameria triandra, oder eine reine Verbindung aus dem Extrakt, die aus der Gruppe Eupomatenoid 6 oder 2-(2,4-Dihydroxyphenyl)-5-propenylbenzofuran ausgewählt ist, und
b) einen lipophilen Extrakt von Mesua ferrea.

2. Pharmazeutische Zubereitungen nach Anspruch 1, wobei das Verhältnis der Extrakte von Krameria zu den Extrakten von Mesua ferrea im Bereich von 1:10 bis 10:1 liegt.

3. Zubereitungen nach Anspruch 2, wobei das Verhältnis 1:1 beträgt.

4. Verwendung
a) eines hydrophilen Extrakts von Krameria sp., vorzugsweise von Krameria triandra, oder einer reinen Verbindung aus diesem Extrakt, die aus der Gruppe Eupomatenoid 6 und 2-(2,4-Dihydroxyphenyl)-5-propenylbenzofuran ausgewählt ist, und
b) eines lipophilen Extrakts von Mesua ferrea,
zur Herstellung eines Arzneimittels mit antimikrobieller Wirkung.

5. Nahrungsmittelzubereitungen oder kosmetische Zubereitungen, die als Konservierungsmittel folgendes enthalten:
a) einen hydrophilen Extrakt von Krameria sp., vorzugsweise von Krameria triandra, oder eine reine Verbindung aus dem Extrakt, die aus der Gruppe Eupomatenoid 6 oder 2-(2,4-Dihydroxyphenyl)-5-propenylbenzofuran ausgewählt ist, und
b) einen lipophilen Extrakt von Mesua ferrea.

6. Verwendung
a) eines hydrophilen Extrakts von Krameria sp., vorzugsweise von Krameria triandra, oder einer reinen Verbindung aus diesem Extrakt, die aus der Gruppe Eupomatenoid 6 oder 2-(2,4-Dihydroxyphenyl)-5-propenylbenzofuran ausgewählt ist, und
b) eines lipophilen Extrakts von Mesua ferrea,
als Konservierungsmittel für Nahrungsmittelzubereitungen oder kosmetische Zubereitungen.

## Revendications

1. Formulations pharmaceutiques ou cosmétiques ayant une activité antimicrobienne, contenant :
a) un extrait hydrophile de *Krameria sp*., de préférence *Krameria triandra*, ou un composé pur provenant dudit extrait, choisi dans la classe formée par l'eupomaténoïde 6 ou le 2-(2,4-dihydroxyphényl)-5-propénylbenzofuranne, et
b) un extrait lipophile de *Mesua ferrea*.

2. Formulations pharmaceutiques selon la revendication 1, dans lesquelles le rapport des extraits de *Krameria* aux extraits de *Mesua ferrea* est compris entre 1:10 et 10:1.

3. Formulations selon la revendication 2, dans lesquelles le rapport est de 1:1.

4. L'utilisation de :
a) un extrait hydrophile de *Krameria sp*., de préférence *Krameria triandra*, ou un composé pur provenant dudit extrait, choisi dans la classe formée par l'eupomaténoïde 6 ou le 2-(2,4-dihydroxyphényl)-5-propénylbenzofuranne, et
b) un extrait lipophile de *Mesua ferrea*,
pour la préparation d'un médicament ayant une activité antimicrobienne.

5. Formulations alimentaires ou cosmétiques contenant, comme conservateurs :
a) un extrait hydrophile de *Krameria sp*., de préférence *Krameria triandra*, ou un composé pur provenant dudit extrait, choisi dans la classe formée par l'eupomaténoïde 6 ou le 2-(2,4-dihydroxyphényl)-5-propénylbenzofuranne, et
b) un extrait lipophile de *Mesua ferrea.*

6. L'utilisation de :
a) un extrait hydrophile *Krameria sp*., de préférence *Krameria triandra*, ou un composé pur provenant dudit extrait, choisi dans la classe formée par l'eupomaténoïde 6 ou le 2-(2,4-dihydroxyphényl)-5-propénylbenzofuranne, et
b) un extrait lipophile de *Mesua ferrea*,
comme conservateur pour formulations alimentaires ou cosmétiques.
